# EUROPEAN PATENT APPLICATION

(11) **EP 2 859 902 A1**
(43) Date of publication of application: **15.04.2015**
(21) Application number: 13800074.0
(22) Date of filing: 03.06.2013
(51) Int. Cl.: A61M 1/00, A61B 17/3203, A61M 3/02

(54) **DUAL CONTAINER-TYPE BALANCED LAVAGE APPARATUS OF THROMBUS REMOVER**

(30) Priority: 08.06.2012 CN 201210187340
(71) Applicant: Li, Guangcheng, Shandong 266555 (CN)
(72) Inventor: ZHANG, Wendi, Qingdao Shandong 266555 (CN); LIU, Yuhan, Qingdao Shandong 266555 (CN); SUI, Songtao, Qingdao Shandong 266555 (CN); LIU, Ping, Qingdao Shandong 266555 (CN); LIN, Jie, Qingdao Shandong 266555 (CN); XUE, Xinxia, Qingdao Shandong 266555 (CN); LIU, Jinxia, Qingdao Shandong 266555 (CN); GUO, Daorui, Qingdao Shandong 266555 (CN); JIANG, Yongjie, Qingdao Shandong 266555 (CN); ZHANG, Wenyong, Qingdao Shandong 266555 (CN); LIU, Jian, Qingdao Shandong 266555 (CN); LI, Guangcheng, Qingdao Shandong 266555 (CN)
(74) Representative: Grünecker Patent- und Rechtsanwälte PartG mbB
(86) International application number: PCT/CN2013/076643
(87) International publication number: WO 2013/182020

(57) **Abstract**

A dual container-type balanced lavage apparatus of a thrombus remover. The apparatus comprises a liquid inlet pipe (1), a liquid outlet pipe (2), pump number one (3), pump number two (4), container number one (5), and container number two (6). Container number one (5) is connected to a high-pressure cavity (7) of a debris aspiration apparatus in the thrombus remover via the liquid inlet pipe (1) and pump number one (3), while a low-pressure cavity (8) of the debris aspiration apparatus is connected to container number two (6) via the liquid outlet pipe (2) and pump number two (4). Arranged on the liquid inlet pipe (1) between pump number one (3) and the high-pressure cavity (7) of the debris aspiration apparatus is flexible pipe section number one (101)for use in cushioning pressure waves generated by pump number one (3). Arranged on the liquid outlet pipe (2) between the low-pressure cavity (8) of the debris aspiration apparatus and pump number two (4) is flexible pipe section number two (201) for use in cushioning pressure waves generated by pump number two (4). The arrangement of flexible pipe sections number one and two (101 and 201) respectively on the liquid inlet pipe (1) and on the liquid outlet pipe (2) allows for use of the flexible pipe walls of each to cushion the pressure waves generated by pumps number one and two (3 and 4), thus facilitating increased operation stability of the entire apparatus.

## Description

### Technical Field

The present invention relates to a thrombus remover in medical apparatus, particularly to a balanced lavage apparatus of a thrombus remover.

### Background Art

At present, thrombus remover is usually used in clinical treatment of cerebral haemorrhage. The above-mentioned thrombus remover comprises a debris aspiration apparatus and a balanced lavage apparatus connected with the debris aspiration apparatus. The above-mentioned balanced lavage apparatus comprises high-pressure pipe, low-pressure pipe, circulating pump, filter and exhaust device. The circulating pump is connected with the high-pressure cavity of the debris aspiration apparatus via the high-pressure pipe, and the exhaust device is connected into the high-pressure pipe,while the low-pressure cavity of the debris aspiration apparatus is connected with the circulating pump via the low-pressure pipe, and the filter is connected into the low-pressure pipe. The following defects exist in the above-mentioned balanced lavage apparatus: firstly, the pressure waves generated by the circulating pump at work could not be well cushioned; secondly, in case of malfunction of the balanced lavage apparatus, the high-pressure pipe could not be closed immediately; thirdly, it is unfavorable for direct observation to the effect of thrombus removal.

Therefore, the above-mentioned balanced lavage apparatus is still to be further improved.

### Contents of Invention

The purpose of the present invention is to solve the technical defects that exist in the balanced lavage apparatus of a thrombus remover in the prior art, and provide a dual container-type balanced lavage apparatus of a thrombus remover.

Technical solutions to achieve the present invention are described as follows:
A dual container-type balanced lavage apparatus of a thrombus remover comprises a liquid inlet pipe, a liquid outlet pipe, pump number one, pump number two, container number one and container number two. Container number one is connected to a high-pressure cavity of a debris aspiration apparatus in the thrombus remover via the liquid inlet pipe and pump number one, while a low-pressure cavity of the debris aspiration apparatus is connected to container number two via the liquid outlet pipe and pump number two.

Arranged on the liquid inlet pipe between the above-mentioned pump number one and the high-pressure cavity of the debris aspiration apparatus is flexible pipe section number one for use in cushioning pressure waves generated by pump number one. Arranged on the liquid outlet pipe between the low-pressure cavity of the debris aspiration apparatus and pump number two is flexible pipe section number two for use in cushioning pressure waves generated by pump number two.

On the above-mentioned flexible pipe section number one, it is configured with gripping valve number one, which can close the liquid inlet pipe immediately in case of malfunction of the balanced lavage apparatus; on the above-mentioned flexible pipe section number two, it is configured with gripping valve number two, which can close the liquid outlet pipe immediately in case of malfunction of the balanced lavage apparatus.

Arranged on the liquid inlet pipe between pump number one and the high-pressure cavity of the debris aspiration apparatus is auxiliary pipe number one, with one end connected into the liquid inlet pipe, and the other end connected with a pressure sensor. Arranged on the liquid outlet pipe between the low-pressure cavity of the debris aspiration apparatus and pump number two is auxiliary pipe number two, with one end connected into the liquid outlet pipe, and the other end connected with a pressure sensor.

The above-mentioned auxiliary pipes number one and two are both connected with a pressure sensor through a pipe connector.

There is a pipe connector arranged between the above-mentioned liquid inlet pipe and container number one; there is also a pipe connector arranged between the above-mentioned liquid outlet pipe and container number two.

The above-mentioned pump number one is a peristaltic type pump station, and there is flexible pipe section number three arranged on the liquid inlet pipe for use in coordinating with pump number one; the above-mentioned pump number two is a peristaltic type pump station, and there is flexible pipe section number four arranged on the liquid outlet pipe for use in coordinating with pump number two.

A section of pipe body of the above-mentioned liquid inlet pipe close to the debris aspiration apparatus is closely parallel together with a section of pipe body of the liquid outlet pipe close to the debris aspiration apparatus.

The power cords for the motor of the above-mentioned debris aspiration apparatus are bunched together, and they are led out of one stalk of conduit; the above-mentioned conduit is closely flush with the above-mentioned a section of pipe body.

The present invention shall have the following beneficial technical effects:
There are flexible pipe sections number one and two respectively arranged on the liquid inlet pipe and on the liquid outlet pipe in the present invention, which, with each flexible pipe walls, can cushion the pressure waves generated by pumps number one and two, thus facilitating increased operation stability of the entire apparatus. In addition, since there are gripping valves number one and two respectively arranged on the flexible pipe sections number one and two, in case of malfunction of the balanced lavage apparatus, the liquid inlet pipe and the liquid outlet pipe can be closed immediately, so as to stop the flow of hydraulic medium and facilitate its increased security. Each pump in the present invention can be peristaltic type pump station, and there are flexible pipe sections number three and four respectively arranged on each pipe for use in coordinating with pumps number one and two. Such technical method can not only facilitate meeting the aseptic requirements of operation, but also facilitate lowering the running and maintenance costs. It particularly facilitates direct observation to the effect of thrombus removal.

### Description of Figures

The present invention is further described as follows in combination with the figures and the specific embodiment:

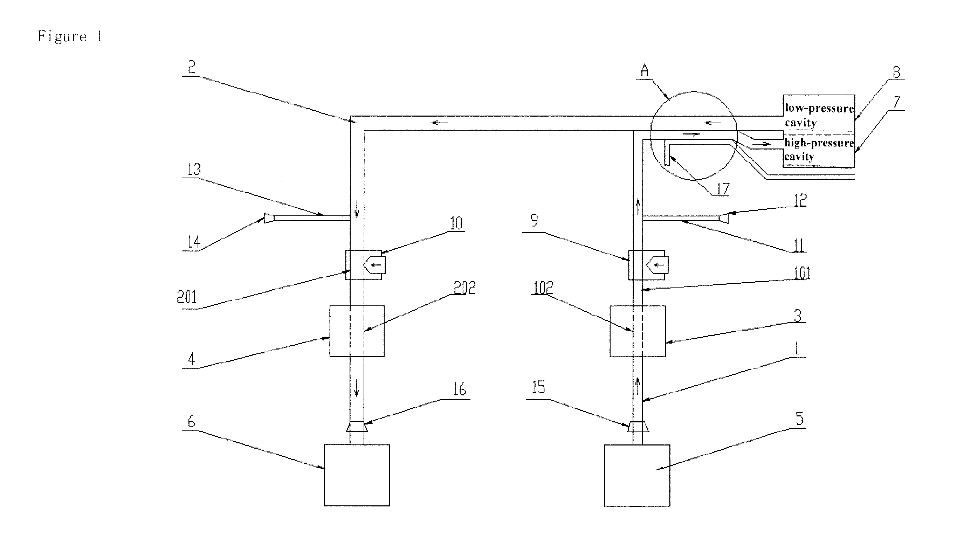

Figure 1 shows a structure diagram of the specific embodiment of the present invention.

### Specific Embodiment

As shown in fig. 1, a dual container-type balanced lavage apparatus of a thrombus remover comprises a liquid inlet pipe (1), a liquid outlet pipe (2), pump number one (3), pump number two (4), container number one (5) and container number two (6). The above-mentioned container number one is connected to a high-pressure cavity (7) of a debris aspiration apparatus in the thrombus remover via the liquid inlet pipe and pump number one, while a low-pressure cavity (8) of the debris aspiration apparatus is connected to container number two via the liquid outlet pipe and pump number two, i.e., container number two is connected to the low-pressure cavity of the debris aspiration apparatus through pump number two and the liquid outlet pipe. Arranged on the liquid inlet pipe between pump number one and the high-pressure cavity of the debris aspiration apparatus is flexible pipe section number one (101), which can cushion the pressure waves generated by pump number one through the flexible pipe walls of flexible pipe section number one. Arranged on the liquid outlet pipe between the low-pressure cavity of the debris aspiration apparatus and pump number two is flexible pipe section number two (201), which can cushion the pressure waves generated by pump number two through the flexible pipe walls of flexible pipe section number two. Gripping valve number one (9) is configured on the above-mentioned flexible pipe section number one, and gripping valve number one is used for closing the liquid inlet pipe immediately in case of malfunction of the balanced lavage apparatus; gripping valve number two (10) is configured on the above-mentioned flexible pipe section, and gripping valve number two is used for closing the liquid outlet pipe immediately in case of malfunction of the balanced lavage apparatus.

In the above embodiment, auxiliary pipe number one (11) can also be arranged on the liquid inlet pipe between pump number one and the high-pressure cavity of the debris aspiration apparatus, with one end connected into the liquid inlet pipe, and the other end connected with a pressure sensor; auxiliary pipe number one can be connected with the pressure sensor through the pipe connector (12). There is auxiliary pipe number two (13) arranged on the liquid outlet pipe between the low-pressure cavity of the debris aspiration apparatus and pump number two, with one end connected into the liquid outlet pipe, and the other end connected with a pressure sensor; The above-mentioned auxiliary pipe number two can be connected with the pressure sensor through the pipe connector (14). In the above embodiment, the pipe connector (15) can be arranged between the liquid inlet pipe and container number one. The pipe connector (16) can be arranged between the liquid outlet pipe and container number two. The above embodiment of pipe connectors shall comparatively facilitate disassembly and butt junction.

In the above embodiment, a section of pipe body of the above-mentioned liquid inlet pipe close to the debris aspiration apparatus can be closely parallel together with a section of pipe body of the liquid outlet pipe close to the debris aspiration apparatus. The power cords for the motor of the debris aspiration apparatus are bunched together, and they are led out of one stalk of conduit (17); the above-mentioned conduit is closely flush with the above-mentioned a section of pipe body. Refer to Part A in the diagram.

In the above embodiment, pump number one is a peristaltic type pump station, and there is flexible pipe section number three (102) arranged on the liquid inlet pipe for use in coordinating with pump number one; the above-mentioned pump number two is a peristaltic pump station, and there is flexible pipe section number four (202) arranged on the liquid outlet pipe for use in coordinating with pump number two.

The working principle of the present invention is briefly described below:
It is divided into the liquid outlet pipe and the liquid inlet pipe according to the flow direction of hydraulic medium.

The pipe is filled with hydraulic medium through the preparatory process of filling. Pump (station) number one and pump (station) number two shall work synchronously, so that pressure difference is produced between pump inlet and pump outlet to actuate flow of liquid. The hydraulic medium firstly enters the liquid inlet pipe from container number one and will be pressurized by pump number one to enter the high-pressure cavity of the debris aspiration apparatus.In the following it will enter the low-pressure cavity of the debris aspiration apparatus through circulation in cranium, then enter the liquid outlet pipe to begin reflux, and it will be delivered into container number two through pressurization by pump number two.

For relevant technical content not iterated in the above embodiment, it can be realized by adopting or drawing experience from the prior art.

It is to be noted that under the guide by the present specification any one of those who skilled in the technique can also easily make some or other change, such as equivalent changes and obvious remodels. The above change shall all be within the protection scope of the present invention.

## Claims

1. A dual container-type balanced lavage apparatus of a thrombus remover, **characterized in that** the apparatus comprises a liquid inlet pipe, a liquid outlet pipe, pump number one, pump number two, container number one and container number two; container number one is connected to a high-pressure cavity of a debris aspiration apparatus in the thrombus remover via the liquid inlet pipe and pump number one, while a low-pressure cavity of the debris aspiration apparatus is connected to container number two via the liquid outlet pipe and pump number two.

2. The mentioned dual container-type balanced lavage apparatus of a thrombus remover according to claim 1, **characterized in that** arranged on the liquid inlet pipe between pump number one and the high-pressure cavity of the debris aspiration apparatus is flexible pipe section number one for use in cushioning the pressure wave generated by pump number one. Arranged on the liquid outlet pipe between the low-pressure cavity of the debris aspiration apparatus and pump number two is flexible pipe section number two for use in cushioning the pressure wave generated by pump number two.

3. The mentioned dual container-type balanced lavage apparatus of a thrombus remover according to claim 2, **characterized in that** on the mentioned flexible pipe section number one, it is configured with gripping valve number one, which can close the liquid inlet pipe immediately in case of malfunction of the balanced lavage apparatus; on the mentioned flexible pipe section number two, it is configured with gripping valve number two, which can close the liquid outlet pipe immediately in case of malfunction of the balanced lavage apparatus.

4. The mentioned dual container-type balanced lavage apparatus of a thrombus remover according to claim 1, **characterized in that** arranged on the mentioned liquid inlet pipe between pump number one and the high-pressure cavity of the debris aspiration apparatus is auxiliary pipe number one with one end connected into the liquid inlet pipe and the other end connected with a pressure sensor; arranged on the liquid outlet pipe between the low-pressure cavity of the debris aspiration apparatus and pump number two is auxiliary pipe number two with one end connected into the liquid outlet pipe, and the other end connected with a pressure sensor.

5. The mentioned dual container-type balanced lavage apparatus of a thrombus remover according to claim 4, **characterized in that** the mentioned auxiliary pipes number one and two are both connected with a pressure sensor through a pipe connector

6. The mentioned dual container-type balanced lavage apparatus of a thrombus remover according to claim 1, **characterized in that** there is a pipe connector arranged between the mentioned liquid inlet pipe and container number one; there is also a pipe connector arranged between the mentioned liquid outlet pipe and container number two.

7. The mentioned dual container-type balanced lavage apparatus of a thrombus remover according to claim 1, **characterized in that** the mentioned pump number one is a peristaltic type pump station, and there is flexible pipe section number three arranged on the liquid inlet pipe for use in coordinating with pump number one; the mentioned pump number two is a peristaltic type pump station, and there is flexible pipe section number four arranged on the liquid outlet pipe for use in coordinating with pump number two.

8. The mentioned dual container-type balanced lavage apparatus of a thrombus remover according to claim 1, **characterized in that** A section of pipe body of the mentioned liquid inlet pipe close to the debris aspiration apparatus is closely parallel together with a section of pipe body of the liquid outlet pipe close to the debris aspiration apparatus.

9. The mentioned dual container-type balanced lavage apparatus of a thrombus remover according to claim 8, **characterized in that** the power cords for the motor of the mentioned debris aspiration apparatus are bunched together, and they are led out of one stalk of conduit; the mentioned conduit is closely flush with the above-mentioned a segment of pipe body.
